# FASCICULE DE BREVET EUROPEEN

(11) **EP 1 343 473 B1**
(45) Date de publication et mention de la délivrance du brevet: **20.12.2006**
(21) Numéro de dépôt: 01270322.9
(22) Date de dépôt: 10.12.2001
(51) Int. Cl.: A61K 9/08, A61K 47/12, A61K 47/40

(54) **COMPOSITION PHARMACEUTIQUE DE DRONEDARONE POUR ADMINISTRATION PARENTERALE**
PHARMAZEUTISCHE DRONEDARON-ZUSAMMENSETZUNG FÜR DIE PARENTERALE VERABREICHUNG
PHARMACEUTICAL DRONEDARONE COMPOSITION FOR PARENTERAL ADMINISTRATION

(30) Priorité: 11.12.2000 FR 0016071
(43) Date de publication de la demande: 17.09.2003
(73) Titulaire: Sanofi-Aventis, 75013 Paris (FR)
(72) Inventeur: BOURRIAGUE-SEVE, Frédérique, F-34190 Ferrieres les Verreries (FR); BREUL, Thierry, F-34110 Frontignan (FR)
(74) Mandataire: Weber, Mathieu
(86) Numéro de dépôt international: PCT/FR2001/003903
(87) Numéro de publication internationale: WO 2002/047660

(56) Documents cités:
- US-A- 5 985 915
- VERDUYN SC; VOS MA; LEUNISSEN HD: "Evaluation of the acute electrophysiologic effects of intraveneous Dronedarone..." JOURNAL OF CARDIOVASCULAR PHARMACOLOGY, vol. 33, no. 2, 1999, pages 212-222, XP001024499
- MANNING A; THISSE V; HODEIGE D: "SR33589, a new amiadarone-like antiarrhythmic agent..." JOURNAL OF CARDIOVASCULAR PHARMACOLOGY, vol. 25, no. 2, 1995, pages 252-261, XP001024497

## Description

La présente invention se rapporte, d'une manière générale, à une nouvelle composition pharmaceutique contenant comme principe actif un dérivé de benzoyl-benzofurane.

En particulier, l'invention concerne une composition pharmaceutique pour administration parentérale contenant comme principe actif le 2-butyl-3-(4-[[3-(dibutylamino)propoxy]]benzoyl)-5-méthanesulfonamido-benzofurane également dénommé dronédarone, ou un de ses sels pharmaceutiquement acceptables de préférence son chlorhydrate.

Ce dérivé de méthanesulfonamido-benzofurane ainsi que ses sels pharmaceutiquement acceptables ont été décrits dans le brevet EP 0471609 de même que ses applications thérapeutiques.

Dans le domaine cardiovasculaire, ce composé s'est montré particulièrement intéressant notamment comme agent antiarythmique.

La dronédarone, sous forme de son chlorhydrate, présente une solubilité limitée en milieu aqueux : à température ambiante, elle est de 0,5 mg/ml à pH = 4,85. Dans la gamme de pH allant de 1,5 à 5, cette solubilité est pratiquement constante puis devient quasi négligeable à des pH supérieurs à 5,5.

Toutefois, la solubilité du chlorhydrate de dronédarone est augmentée en présence d'un tampon phosphate monosodique (NaH₂PO₄): à température ambiante, elle varie de 2 mg/ml à 2,25 mg/ml dans la gamme de pH allant de 1,5 à 5 tandis qu'elle diminue brutalement pour devenir quasi nulle à pH = 5,5.

Des tests effectués sur rat durant plusieurs jours avec une formulation aqueuse contenant 1,065 mg/ml de chlorhydrate de dronédarone en présence d'un tampon phosphate ont montré cependant une mauvaise tolérance locale de cette composition au contraire d'une formulation identique exempte de ce chlorhydrate.

D'autre part, la dronédarone est douée de propriétés tensio-actives ioniques puissantes provoquant, en solution aqueuse, une diminution marquée de la tension superficielle. Ce caractère tensio-actif se révèle d'ailleurs aussi puissant en présence qu'en l'absence d'un tampon phosphate et peut être tenu comme partiellement responsable d'une auto-agrégation des molécules de dronédarone en solution aqueuse. En fait, cette organisation supramoléculaire en solution est due à la formation de dimères disposés tête-bêche et empilés en couches superposées. Ces dimères, lors de leur administration par injection, provoquent la rupture de la membrane cellulaire et, par voie de conséquence, l'apparition d'inflammations au site d'injection. De façon générale, ces propriétés tensio-actives de la dronédarone peuvent être considérées comme responsables d'une mauvaise tolérance de la composition les contenant.

On a rapporté, dans le brevet US n° 4727064, que des solutions aqueuses de dérivés de cyclodextrine notamment l'hydroxypropyl-β-cyclodextrine sont capables d'augmenter la solubilité de principes actifs médicamenteux, ces solutions de dérivés de cyclodextrine ne provoquant pas d'irritation locale. Toutefois, aucune indication n'y figure au sujet de la tolérance locale de telles solutions de dérivés de β-cyclodextrine contenant également un principe actif médicamenteux.

Dans le cadre de l'invention, on a émis l'hypothèse que des dérivés de cyclodextrine présents dans une formulation aqueuse de dronédarone, sous forme par exemple de son chlorhydrate, pourraient améliorer la tolérance de ce principe actif lors d'une administration parentérale.

Dans cette optique, des tests ont été effectués sur rat durant plusieurs jours avec une formulation aqueuse contenant 1,065 mg/ml de chlorhydrate de dronédarone en présence d'un dérivé de cyclodextrine en l'occurrence l'hydroxypropyl-β-cyclodextrine. Toutefois l'administration d'une telle composition a montré une intolérance marquée au site d'injection alors qu'une formulation identique, cependant exempte de chlorhydrate de dronédarone, s'est révélée bien tolérée.

La recherche d'une formulation injectable de dronédarone, de préférence sous forme d'un de ses sels pharmaceutiquement acceptables tel que son chlorhydrate, qui soit à la fois suffisamment concentrée pour une utilisation thérapeutique et dépourvue des désavantages rapportés précédemment reste par conséquent d'un intérêt incontestable.

Or, on a maintenant découvert, de manière surprenante selon l'invention, qu'il est possible de disposer de solutions aqueuses de dronédarone, en particulier sous forme de son chlorhydrate, à la fois concentrées et pourvues d'une tolérance acceptable lors de leur administration.

En fait, on a pu mettre en évidence que des dérivés de β-cyclodextrine, lorsqu'ils sont associés à un milieu tampon approprié, sont capables d'augmenter la solubilité de la dronédarone ou de ses sels pharmaceutiquement acceptables et de prévenir leur auto-agrégation.

En conséquence, l'invention concerne une composition pharmaceutique pour administration parentérale comprenant :
- la dronédarone ou un de ses sels pharmaceutiquement acceptables en tant que principe actif,
- une solution tampon physiologiquement acceptable capable de maintenir le pH de la composition entre 3 et 5,
- un dérivé hydrosoluble de β-cyclodextrine physiologiquement acceptable.

Dans la suite de la description de même que dans les revendications et sauf indications contraires, les pourcentages des différents constituants formant les compositions de l'invention à savoir le principe actif, le milieu tampon, le dérivé de β-cyclodextrine ou tout autre ingrédient supplémentaire, expriment tous des proportions pondérales à savoir des % en poids de la composition finale.

La dronédarone, préférentiellement sous forme d'un de ses sels pharmaceutiquement acceptables par exemple le chlorhydrate, est présente dans les compositions de l'invention à raison de 0,01 % à 4 % par exemple à raison de 0,1 % à 0,8 %. Des compositions préférées contiennent toutefois de 0,4 % à 0,8 % de dronédarone ou d'un de ses sels pharmaceutiquement acceptables.

En général, on choisit la solution tampon parmi des solutions aqueuses physiologiquement acceptables capables à la fois de dissoudre le principe actif et de maintenir le pH des compositions entre 3 et 5.

Ainsi, des solutions tampons utilisables dans le cadre de l'invention peuvent être par exemple des solutions aqueuses contenant un système tampon choisi parmi les suivants :
- acide acétique/acétate de métal alcalin,
- acide fumarique/fumarate de métal alcalin,
- acide succinique/succinate de métal alcalin,
- acide citrique/citrate de métal alcalin,
- acide tartrique/tartrate de métal alcalin,
- acide lactique/lactate de métal alcalin,
- acide maléique/maléate de métal alcalin,
- acide méthanesulfonique/méthanesulfonate de métal alcalin,
- phosphate monométallique alcalin,
le métal alcalin dans chacun des sels ci-dessus étant par exemple le sodium ou le potassium.

A titre préférentiel, on utilise comme tampon un phosphate monométallique alcalin par exemple le phosphate monosodique ou monopotassique. Ce tampon est capable de maintenir le pH de la composition à 4,5, pH à partir duquel une augmentation de celui-ci provoque une diminution drastique de la solubilité aqueuse de la dronédarone en particulier de son chlorhydrate.

Dans la majorité des cas, la force ionique de la solution tampon sera comprise entre 0,005 molaire et 0,5 molaire de préférence entre 0,01 et 0,2 molaire, par exemple de 0,05 à 0,15 molaire. Au-delà de 0,5 molaire, la concentration en sels dans le milieu risque de perturber la stabilité des compositions de l'invention et d'entraîner une hypertonie des formulations alors qu'à une concentration inférieure à 0,005 molaire l'effet tampon devient inexistant.

A titre d'exemple, on peut citer des solutions aqueuses tampons 0,05 à 0,15 molaire en phosphate monométallique alcalin par exemple, monosodique ou monopotassique.

Quant au dérivé hydrosoluble de β-cyclodextrine, on le choisit parmi des composés de ce type physiologiquement acceptables, ce dérivé étant généralement plus soluble dans l'eau que la β-cyclodextrine elle-même. Cette dernière s'est d'ailleurs révélée inutilisable en raison de sa faible solubilité aqueuse et de sa toxicité.

A titre d'exemple de dérivés de β-cyclodextrine utilisables dans les compositions de l'invention, on peut citer le dérivé sulfobutyl éther de β-cyclodextrine, la diméthyl-β-cyclodextrine, la triméthyl-β-cyclodextrine ainsi que leurs mélanges. Toutefois, on préfère utiliser l'hydroxypropyl-β-cyclodextrine.

En général, le dérivé de β-cyclodextrine est incorporé dans les compositions parentérales à raison de 0,5 % à 50 % de préférence de 1 % à 10 %, par exemple 5 % et de 5 à 20 fois le poids de dronédarone ou d'un de ses sels pharmaceutiquement acceptables, de préférence de 10 à 15 fois ce poids et plus particulièrement de 12 à 13 fois ce poids.
Comme indiqué précédemment, les dérivés de β-cyclodextrine ci-dessus, lorsqu'ils sont associés à un tampon approprié, par exemple un tampon phosphate, dans les compositions de l'invention, permettent notamment d'accroître la solubilité aqueuse de la dronédarone ou de ses sels pharmaceutiquement acceptables. Dans le cas par exemple du chlorhydrate de dronédarone, la solubilité peut être augmentée dans ces conditions jusqu'à environ 6,5 mg/ml en présence d'un tampon phosphate 100 mmolaire et de 5 % d'hydroxypropyl-β-cyclodextrine.
En outre, ces dérivés de β-cyclodextrine, en particulier l'hydroxypropyl-β-cyclodextrine, se sont révélés aptes à prévenir l'auto-association des molécules de dronédarone ou de ses sels pharmaceutiquement acceptables, par formation d'un complexe d'encapsulation de ce principe actif. Ce complexe d'encapsulation moléculaire s'est ensuite révélé capable de traverser la membrane cellulaire en la maintenant intacte et, par conséquent, de remédier aux problèmes d'intolérance locale mis en évidence avec des compositions contenant soit un tampon approprié soit un dérivé de β-cyclodextrine.
Si nécessaire, les compositions de l'invention peuvent comprendre un ou plusieurs ingrédients supplémentaires notamment un agent conservateur ou protecteur tel qu'un bactéricide ou un composé permettant de maintenir l'isotonicité de la composition tel que le mannitol. Les compositions de l'invention ainsi formées, se caractérisent par une stabilité importante qui permet à la fois de les conserver de manière durable et de les utiliser efficacement pour une administration par injection.

Les compositions pharmaceutiques de l'invention peuvent être obtenues de manière classique en chauffant à une température de 50° C à 60° C, de préférence à 55° C, un milieu aqueux formé du système tampon approprié, du dérivé soluble de β-cyclodextrine et des éventuels excipients supplémentaires puis en y introduisant, à cette température, le principe actif.

Les caractéristiques et avantages des compositions selon l'invention apparaîtront à la lumière de la description ci-après à partir de compositions données à titre d'exemples.

### I. Tolérance locale chez l'animal

Des tests ont été pratiqués en vue d'évaluer la tolérance locale de différentes compositions aqueuses contenant le chlorhydrate de dronédarone ou uniquement le véhicule correspondant à titre de placebo.

A cet effet, on a administré journellement, par voie intraveineuse à des lots de 5 rats mâles pendant 5 jours, une dose d'une des compositions aqueuses ci-dessous. On a pratiqué l'injection dans la veine caudale, de préférence, en zone distale, puis en zone médiane et enfin en zone proximale.
On a alors sacrifié les animaux au 8ème jour et on a prélevé les queues en vue d'un éventuel examen microscopique.

On a obtenu les résultats suivants :

### a) Compositions à 0,1 % de dronédarone base

• Dose administrée : 5 ml/kg soit 5 mg/kg/jour
• Compositions testées

| | COMPOSITION (en mg) | | | | | |
|---|---|---|---|---|---|---|
| INGREDIENTS | A | B | C | D | E | F |
| Chlorhydrate de dronédarone (correspondant à 1 mg de dronédarone base) | 1,065 | - | 1,065 | - | 1,065 | - |
| Hydroxypropyl-β-cyclodextrine | 50 | 50 | 50 | 50 | - | - |
| Phosphate monosodique anhydre | 12 | 12 | - | - | 12 | 12 |
| Mannitol apyrogène | 8 | 8 | 45 | 45 | 20 | 20 |

Ces ingrédients sont ajoutés à la quantité suffisante d'eau pour obtenir un volume de 1 ml.

Les compositions A, B, E et F ont un pH de 4,5 et les compositions C et D, un pH compris entre 4,5 et 6,5.
1) Composition A (ou composition contenant un dérivé de β-cyclodextrine et un tampon phosphate)
   Aucun signe clinique local n'a été noté.
   La tolérance locale de cette formulation peut être considérée comme très bonne.
2) Compositions B. D et F (ou compositions placebo)
   Aucun signe clinique local n'a été enregistré.
3) Composition C (ou composition contenant un dérivé de β-cyclodextrine mais pas de tampon phosphate)
   Après une seule injection, la queue de tous les animaux a présenté des rougeurs locales et était de consistance dure. A partir de la deuxième injection, les suivantes se sont révélées douloureuses et ont dû être effectuées dans le segment médian puis proximal (base) de la queue.
   Compte tenu de l'état de la queue, il n'a plus été possible ensuite de réaliser des injections. Ainsi, 2 animaux n'ont pu recevoir que 3 injections, 2 autres n'en ont reçu que 4 tandis qu'un seul a pu être traité durant les 5 jours. Au 5ème jour, les queues de 4 animaux sont apparues noires (nécrose probable).
   L'intolérance locale de cette composition est considérée comme sévère.
4) Composition E (ou composition contenant un tampon phosphate mais pas de dérivé de β-cyclodextrine)
   Après 1 ou 3 injections, la queue des animaux a présenté des rougeurs locales sur toute la longueur. Au 4ème jour, 2 animaux ont dû recevoir l'injection dans le segment médian (et non plus distal). Au 5ème jour, les difficultés techniques dues au mauvais état des queues ont empêché le traitement de 4 animaux et la queue de tous les animaux était de consistance dure.

L'intolérance locale de cette formulation est considérée comme marquée.

En conclusion, l'ensemblé de ces résultats montre clairement que parmi les compositions contenant le chlorhydrate de dronédarone, seule la formulation faisant appel à la fois à un tampon et à un dérivé de β-cyclodextrine a montré une tolérance locale satisfaisante.

### b) Compositions à 0.4 % de dronédarone base

• Dose administrée : 2 ml/kg soit 2 mg/kg/jour
• Compositions testées

| | COMPOSITION (en mg) | | | |
|---|---|---|---|---|
| INGREDIENTS | G | H | I | J |
| Chlorhydrate de dronédarone (correspondant à 4 mg de dronédarone base) | 4,260 | - | 4,260 | - |
| Hydroxypropyl-β-cyclodextrine | 50 | 50 | - | - |
| Mélange de dérivés méthylés de β-cyclodextrine | - | - | 50 | 50 |
| Phosphate monosodique anhydre | 12 | 12 | 12 | 12 |
| Mannitol apyrogène | 8 | 8 | 8 | 8 |

Ces ingrédients sont ajoutés à la quantité suffisante d'eau pour obtenir un volume de 1 ml.

Les quatre compositions ont un pH de 4,5.
1) Compositions G et I (ou compositions contenant un dérivé de β-cyclodextrine et un tampon phosphate)
   Aucun signe clinique local n'a été noté.
2) Compositions H et J (ou compositions placebo)
   Aucun signe clinique local n'a été enregistré.
   La tolérance locale de ces formulations peut être considérée comme très bonne et équivalente.

Ces résultats montrent à nouveau l'excellente tolérance locale des compositions contenant le chlorhydrate de dronédarone lorsque celles-ci incluent à la fois un tampon approprié et un dérivé de β-cyclodextrine.

### II. Tolérance locale chez l'homme

Des tests comparatifs ont été effectués en vue d'évaluer la tolérance locale de deux formulations de chlorhydrate de dronédarone, l'une contenant un tampon phosphate mais pas de dérivé de β-cyclodextrine en l'occurrence la Composition E, l'autre incluant ces deux composés à savoir :

| INGREDIENTS | COMPOSITION K (en mg) |
|---|---|
| Chlorhydate de dronédarone (correspondant à 4 mg de dronédarone base) | 4,26 |
| Mannitol apyrogène | 8,0 |
| Hydroxypropyl-β-cyclodextrine | 50,0 |
| Phosphate monosodique dihydraté | 15,6 |

Ces ingrédients sont ajoutés à la quantité suffisante d'eau pour obtenir un volume de 1 ml.
A) Dans une première série de tests, on a utilisé 28 sujets sains de sexe masculin répartis en 7 groupes de 4 sujets :
   - 3 sujets de chaque groupe ont reçu, à partir de la Composition E administrée par perfusion dans la veine antécubitale, une dose de chlorhydrate de dronédarone correspondant à 5,10, 20 ou 40 mg de dronédarone base et ce, en 30 minutes ou une dose de chlorhydrate de dronédarone correspondant à 40, 60 ou 80 mg de dronédarone base et ce, en 60 minutes, 1 sujet par groupe ayant reçu le placebo également par perfusion. Cette Composition E a été utilisée après dilution dans une solution à 5 % de dextrose de manière à obtenir pour chacun des tests, une concentration en principe actif de 0,333 mg/ml. Par conséquent, le volume de perfusion et la vitesse d'administration par minute a changé pour chaque dose testée.

   A la suite de ces perfusions, 2 sujets ayant reçu leur dose en 30 minutes ont présenté une phlébite qui a disparu en laissant persister un durcissement de la veine toujours présent après 8 jours.
   De même, 3 sujets ayant reçu leur dose en 60 minutes ont présenté une veinite. Celle-ci a disparu mais a laissé un durcissement de la veine encore présent après 8 jours.
   Aucun des sujets ayant reçu le placebo n'a manifesté de réaction au site de perfusion.
B) Dans une deuxième série de tests, on a utilisé 32 sujets sains de sexe masculin répartis en 5 groupes :
   - 3 sujets de 2 groupes ont reçu, à partir de la Composition K administrée par perfusion dans la veine antécubitale, une dose de chlorhydrate de dronédarone correspondant à 10 ou 20 mg de dronédarone base et ce, en 30 minutes, 1 sujet par groupe ayant reçu le placebo également par perfusion.
   - 6 sujets de 3 groupes ont reçu, à partir de la Composition K administrée par perfusion dans la veine antécubitale, une dose de chlorhydrate de dronédarone correspondant à 40, 60 ou 80 mg de dronédarone base et ce, en 30 minutes, 2 sujets par groupe ayant reçu le placebo également par perfusion.
   Cette Composition K a été utilisée après dilution dans une solution à 5 % de dextrose. En outre, on a fixé le volume de perfusion à 120 ml et la vitesse d'administration à 4 ml/minute.

Par conséquent, la concentration en chlorhydrate de dronédarone a augmenté dans le liquide de perfusion selon la dose de principe actif à administrer passant de 0,083 mg/ml pour une dose de 10 mg à 0,666 mg/ml pour une dose de 80 mg.

A la suite de ces perfusions, la tolérance locale est apparue meilleure qu'avec la Composition E puisqu'aucune phlébite ni veinite n'a été enregistrée à chacune des doses testées.

Comparativement à la Composition E, les résultats enregistrés avec la Composition K sont particulièrement remarquables aux doses de 40, 60 et 80 mg puisque la durée de perfusion a été plus courte (30 minutes au lieu de 60 minutes pour la Composition E) et la concentration du liquide de perfusion administré plus élevée.

En effet, à la dose de 80 mg, la concentration en chlorhydrate de dronédarone dans la Composition K perfusée était de 0,666 mg/ml au lieu de 0,333 mg/ml le cas de la Composition E.

Ces résultats confirment, sur être humain, la très bonne tolérance des compositions contenant le chlorhydrate de dronédarone, un tampon approprié et un dérivé de β-cyclodextrine par rapport aux compositions analogues dépourvues d'un tampon.

Les exemples non limitatifs suivants illustrent l'invention.

### EXEMPLE 1

### Composition injectable de chlorhydrate de dronédarone

On prépare une composition pharmaceutique injectable répondant à la formulation suivante :

| | |
|---|---|
| Chlorhydrate de dronédarone (correspondant à 4 mg de dronédarone base) | 4,26 mg |
| Hydroxypropyl-β-cyclodextrine | 50,00 mg |
| Phosphate monosodique dihydraté | 15,60 mg |
| Mannitol | 8,00 mg |
| Eau | quantité suffisante pour 1 ml |
| (pour préparation injectable) | |

par application du procédé décrit ci-dessous.

On solubilise 50 mg d'hydroxypropyl-β-cyclodextrine, 15,6 mg de phosphate monosodique dihydraté et 8 mg de mannitol dans 75 % de l'eau pour préparation injectable totale. On chauffe à 55°C la solution obtenue puis, sous agitation magnétique, on introduit 4,26 mg de chlorhydrate de dronédarone. On poursuit l'agitation magnétique durant 30 minutes puis on complète la solution jusqu'à 100 % du volume final avec de l'eau pour préparation injectable. On filtre la solution de chlorhydrate de dronédarone sur membrane de porosité 0,22 mm et on vérifie la limpidité de cette solution, le pH et l'osmolalité.

On a obtenu les résultats suivants :
Turbidité : 1,1 NTU (unité de turbidité néphélométrique)
pH : 4,5
Osmolalité : 311 mosmol./kg

La solution ainsi obtenue est limpide et isotonique. Elle peut être portée en autoclave à 121° C pendant 35 minutes. Elle est stable pendant 6 mois à 5° C, 25° C et 40° C et diluable en solution à 5 % de glucose et 0,9 % de chlorure de sodium.

### EXEMPLES 2 à 4

En suivant la même méthode qu'à l'Exemple 1, on a préparé les compositions suivantes :

| | | |
|---|---|---|
| Ex.2 | Chlorhydrate de dronédarone (correspondant | |
| | à 4 mg de dronédarone base) | 4,26 mg |
| | Hydroxypropyl-β-cyclodextrine | 50,00 mg |
| | Phosphate monosodique anhydre | 12,00 mg |
| | Mannitol | 8,00 mg |
| | Eau | quantité suffisante pour 1 ml |
| | (pour préparation injectable) | |
| Ex. 3 | Chlorhydrate de dronédarone (correspondant | |
| | à 1 mg de dronédarone base) | 1,065 mg |
| | Hydroxypropyl-β-cyclodextrine | 50,00 mg |
| | Phosphate monosodique anhydre 12,00 mg | |
| | Mannitol | 8,00 mg |
| | Eau | quantité suffisante pour 1 ml |
| | (pour préparation injectable) | |
| Ex. 4 | Chlorhydrate de dronédarone (correspondant | |
| | à 4 mg de dronédarone de base) | 4,26 mg |
| | Mélange de dérivés méthylés de | |
| | β-cyclodextrine | 50,00 mg |
| | Mannitol | 8,00 mg |
| | Eau | quantité suffisante pour 1 ml |
| | (pour préparation injectable) | |

### EXEMPLE 5

### Unité de dosage pour administration injectable de chlorhydrate de dronédarone

On stérilise en autoclave à 121° C pendant 35 minutes, une ampoule en verre d'un volume total de 3 ml contenant 1 ml d'une composition de chlorhydrate de dronédarone telle que préparée à l'Exemple 1.

On scelle alors l'ampoule sous aseptie de manière à constituer une unité de dosage contenant 4,26 mg de chlorhydrate de dronédarone équivalant à 0,4 % de dronédarone base.

## Revendications

1. Composition pharmaceutique pour administration parentérale, **caractérisée en ce qu'**elle comprend :
• la dronédarone ou un de ses sels pharmaceutiquement acceptables en tant que principe actif,
• une solution tampon physiologiquement acceptable capable de maintenir le pH de la composition entre 3 et 5,
• un dérivé hydrosoluble de β-cyclodextrine physiologiquement acceptable.

2. Composition pharmaceutique selon la Revendication 1, **caractérisée en ce qu'**elle contient de 0,01 % à 4 % de principe actif.

3. Composition pharmaceutique selon la Revendication 2, **caractérisée en ce qu'**elle contient de 0,4 à 0,8 % de principe actif.

4. Composition pharmaceutique selon une des Revendications 1 à 3, **caractérisée en ce que** la solution tampon est une solution aqueuse contenant un système tampon choisi parmi les suivants :
• acide acétique/acétate de métal alcalin
• acide fumarique/fumarate de métal alcalin
• acide succinique/succinate de métal alcalin
• acide citrique/citrate de métal alcalin
• acide tartrique/tartrate de métal alcalin
• acide lactique/lactate de métal alcalin
• acide maléique/maléate de métal alcalin
• acide méthanesulfonique/méthanesulfonate de métal alcalin
• phosphate monométallique alcalin

5. Composition pharmaceutique selon la Revendication 4, **caractérisée en ce que** le système tampon est un phosphate monométallique alcalin.

6. Composition pharmaceutique selon la Revendication 4, **caractérisée en ce que** la solution tampon de phosphate monométallique alcalin maintient le pH à 4,5.

7. Composition pharmaceutique selon une des Revendications 1 à 6, **caractérisée en ce que** la force ionique de la solution tampon est comprise entre 0,005 molaire et 0,5 molaire.

8. Composition pharmaceutique selon la Revendication 7, **caractérisée en ce que** la force ionique de la solution tampon est comprise entre 0,01 molaire et 0,2 molaire.

9. Composition pharmaceutique selon la Revendication 7 ou 8, **caractérisée en ce que** la force ionique de la solution tampon est comprise entre 0,05 et 0,15 molaire.

10. Composition pharmaceutique selon les Revendications 1 à 9, **caractérisée en ce que** la solution tampon est une solution aqueuse 0,05 à 0,15 molaire en phosphate monométallique alcalin.

11. Composition pharmaceutique selon la Revendication 4, 5, 6 ou 10, **caractérisée en ce que** le métal alcalin est le sodium ou le potassium.

12. Composition pharmaceutique selon une des Revendications 1 à 11, **caractérisée en ce que** le dérivé hydrosoluble de β-cyclodextrine est choisi parmi l'hydroxypropyl-β-cyclodextrine, le dérivé sulfobutyl éther de β-cyclodextrine, la diméthyl-β-cyclodextrine, la triméthyl-β-cyclodextrine ainsi que leurs mélanges.

13. Composition pharmaceutique selon une des Revendications 1 à 12, **caractérisée en ce que** le dérivé hydrosoluble de β-cyclodextrine est l'hydroxypropyl-β-cyclodextrine.

14. Composition pharmaceutique selon une des Revendications 1 à 13, **caractérisée en ce que** le dérivé hydrosoluble de β-cyclodextrine est présent à raison de 0,5 à 50 %.

15. Composition pharmaceutique selon une des Revendications 1 à 14, **caractérisée en ce que** le dérivé hydrosoluble de β-cydodextrine est présent à raison de 5 à 20 fois le poids de dronédarone ou d'un de ses sels pharmaceutiquement acceptables.

16. Composition pharmaceutique selon la Revendication 15, **caractérisée en ce que** le dérivé hydrosoluble de β-cyclodextrine est présent à raison de 12 à 13 fois le poids de dronédarone ou d'un de ses sels pharmaceutiquement acceptables.

17. Composition pharmaceutique selon une des Revendications 1 à 16, **caractérisée en ce qu'**elle contient en outre un agent conservateur ou protecteur.

18. Composition pharmaceutique selon une des Revendications 1 à 17, **caractérisée en ce qu'**elle contient en outre un composé permettant de maintenir l'isotonicité.

19. Composition pharmaceutique selon une des Revendications 1 à 18, **caractérisée en ce que** le principe actif est le chlorhydrate de dronédarone.

## Claims

1. Pharmaceutical composition for parenteral administration, **characterized in that** it comprises:
• dronedarone or one of its pharmaceutically acceptable,salts as active principle,
• a physiologically acceptable buffer solution capable of maintaining the pH of the composition between 3 and 5,
• a physiologically acceptable water-soluble β-cyclodextrin derivative.

2. Pharmaceutical composition according to Claim 1, **characterized in that** it comprises from 0.01% to 4% of active principle.

3. Pharmaceutical composition according to Claim 2, **characterized in that** it comprises from 0.4 to 0.8% of active principle.

4. Pharmaceutical composition according to one of Claims 1 to 3, **characterized in that** the buffer solution is an aqueous solution comprising a buffer system chosen from the following:
• acetic acid/alkali metal acetate,
• fumaric acid/alkali metal fumarate,
• succinic acid/alkali metal succinate,
• citric acid/alkali metal citrate,
• tartaric acid/alkali metal tartrate,
• lactic acid/alkali metal lactate,
• maleic acid/alkali metal maleate,
• methanesulphonic acid/alkali metal methanesulphonate,
• monoalkali metal phosphate.

5. Pharmaceutical composition according to Claim 4, **characterized in that** the buffer system is a monoalkali metal phosphate.

6. Pharmaceutical composition according to Claim 4, **characterized in that** the monoalkali metal phosphate buffer solution maintains the pH at 4.5.

7. Pharmaceutical composition according to one of Claims 1 to 6, **characterized in that** the ionic strength of the buffer solution is comprised between 0.005 molar and 0.5 molar.

8. Pharmaceutical composition according to Claim 7, **characterized in that** the ionic strength of the buffer solution is comprised between 0.01 molar and 0.2 molar.

9. Pharmaceutical composition according to Claim 7 or 8, **characterized in that** the ionic strength of the buffer solution is comprised between 0.05 and 0.15 molar.

10. Pharmaceutical composition according to Claims 1 to 9, **characterized in that** the buffer solution is a 0.05 to 0.15 molar aqueous solution of monoalkali metal phosphate.

11. Pharmaceutical composition according to Claim 4, 5, 6 or 10, **characterized in that** the alkali metal is sodium or potassium.

12. Pharmaceutical composition according to one of Claims 1 to 11, **characterized in that** the water-soluble β-cyclodextrin derivative is chosen from hydroxypropyl-β-cyclodextrin, the sulphobutyl ether derivative of β-cyclodextrin, dimethyl-β-cyclodextrin, trimethyl-β-cyclodextrin, and their mixtures.

13. Pharmaceutical composition according to one of Claims 1 to 12, **characterized in that** the water-soluble β-cyclodextrin derivative is hydroxypropyl-β-cyclodextrin.

14. Pharmaceutical composition according to one of Claims 1 to 13, **characterized in that** the water-soluble β-cyclodextrin derivative is present in a proportion of 0.5 to 50%.

15. Pharmaceutical composition according to one of Claims 1 to 14, **characterized in that** the water-soluble β-cyclodextrin derivative is present in a proportion of 5 to 20 times the weight of dronedarone or of one of its pharmaceutically acceptable salts.

16. Pharmaceutical composition according to Claim 15, **characterized in that** the water-soluble β-cyclodextrin derivative is present in a proportion of 12 to 13 times the weight of dronedarone or of one of its pharmaceutically acceptable salts.

17. Pharmaceutical composition according to one of Claims 1 to 16, **characterized in that** it additionally comprises a preservative or protective agent.

18. Pharmaceutical composition according to one of Claims 1 to 17, **characterized in that** it additionally comprises a compound which makes it possible to maintain the isotonicity.

19. Pharmaceutical composition according to one of Claims 1 to 18, **characterized in that** the active principle is dronedarone hydrochloride.

## Patentansprüche

1. Pharmazeutische Zusammensetzung zur parenteralen Verabreichung, **dadurch gekennzeichnet, dass** sie Folgendes umfasst:
• Dronedaron oder eines seiner pharmazeutisch verträglichen Salze als Wirkstoff,
• eine physiologisch verträgliche Pufferlösung, die in der Lage ist, den pH der Zusammensetzung zwischen 3 und 5 zu halten,
• ein physiologisch verträgliches, wasserlösliches β-Cyclodextrin-Derivat.

2. Pharmazeutische Zusammensetzung nach Anspruch 1, **dadurch gekennzeichnet, dass** sie 0,01% bis 4% Wirkstoff enthält.

3. Pharmazeutische Zusammensetzung nach Anspruch 2, **dadurch gekennzeichnet, dass** sie 0,4% bis 0,8% Wirkstoff enthält.

4. Pharmazeutische Zusammensetzung nach einem der Ansprüche 1 bis 3, **dadurch gekennzeichnet, dass** die Pufferlösung eine wässrige Lösung ist, die ein Puffersystem enthält, das aus den Folgenden ausgewählt ist:
• Essigsäure/Alkalimetallacetat
• Fumarsäure/Alkalimetallfumarat
• Bernsteinsäure/Alkalimetallsuccinat
• Citronensäure/Alkalimetallcitrat
• Weinsäure/Alkalimetalltartrat
• Milchsäure/Alkalimetalllactat
• Maleinsäure/Alkalimetallmaleat
• Methansulfonsäure/Alkalimetallmethansulfonat
• Monoalkalimetallphosphat

5. Pharmazeutische Zusammensetzung nach Anspruch 4, **dadurch gekennzeichnet, dass** das Puffersystem ein Monoalkalimetallphosphat ist.

6. Pharmazeutische Zusammensetzung nach Anspruch 4, **dadurch gekennzeichnet, dass** die Monoalkalimetallphosphat-Pufferlösung den pH bei 4,5 hält.

7. Pharmazeutische Zusammensetzung nach einem der Ansprüche 1 bis 6, **dadurch gekennzeichnet, dass** die Ionenstärke der Pufferlösung zwischen 0,005 molar und 0,5 molar liegt.

8. Pharmazeutische Zusammensetzung nach Anspruch 7, **dadurch gekennzeichnet, dass** die Ionenstärke der Pufferlösung zwischen 0,01 molar und 0,2 molar liegt.

9. Pharmazeutische Zusammensetzung nach Anspruch 7 oder 8, **dadurch gekennzeichnet, dass** die Ionenstärke der Pufferlösung zwischen 0,05 molar und 0,15 molar liegt.

10. Pharmazeutische Zusammensetzung nach den Ansprüchen 1 bis 9, **dadurch gekennzeichnet, dass** die Pufferlösung eine wässrige Lösung ist, die 0,05 bis 0,15 molar an Monoalkalimetallphosphat ist.

11. Pharmazeutische Zusammensetzung nach Anspruch 4, 5, 6 oder 10, **dadurch gekennzeichnet, dass** das Alkalimetall Natrium oder Kalium ist.

12. Pharmazeutische Zusammensetzung nach einem der Ansprüche 1 bis 11, **dadurch gekennzeichnet, dass** das wasserlösliche β-Cyclodextrin-Derivat aus Hydroxypropyl-β-cyclodextrin, dem Sulfobutylether-Derivat von β-Cyclodextrin, Dimethyl-β-cyclodextrin, Trimethyl-β-cyclodextrin sowie deren Gemischen ausgewählt ist.

13. Pharmazeutische Zusammensetzung nach einem der Ansprüche 1 bis 12, **dadurch gekennzeichnet, dass** es sich bei dem wasserlöslichen β-Cyclodextrin-Derivat um Hydroxypropyl-β-cyclodextrin handelt.

14. Pharmazeutische Zusammensetzung nach einem der Ansprüche 1 bis 13, **dadurch gekennzeichnet, dass** das wasserlösliche β-Cyclodextrin-Derivat zu 0,5 bis 50% vorliegt.

15. Pharmazeutische Zusammensetzung nach einem der Ansprüche 1 bis 14, **dadurch gekennzeichnet, dass** das wasserlösliche β-Cyclodextrin-Derivat in dem 5- bis 20-fachen des Gewichts von Dronedaron oder einem seiner pharmazeutisch verträglichen Salze vorliegt.

16. Pharmazeutische Zusammensetzung nach Anspruch 15, **dadurch gekennzeichnet, dass** das wasserlösliche β-Cyclodextrin-Derivat in dem 12- bis 13-fachen des Gewichts von Dronedaron oder einem seiner pharmazeutisch verträglichen Salze vorliegt.

17. Pharmazeutische Zusammensetzung nach einem der Ansprüche 1 bis 16, **dadurch gekennzeichnet, dass** sie außerdem ein Konservierungs- oder Schutzmittel enthält.

18. Pharmazeutische Zusammensetzung nach einem der Ansprüche 1 bis 17, **dadurch gekennzeichnet, dass** sie außerdem eine Verbindung enthält, die es gestattet, die Isotonizität aufrechtzuerhalten.

19. Pharmazeutische Zusammensetzung nach einem der Ansprüche 1 bis 18, **dadurch gekennzeichnet, dass** der Wirkstoff Dronedaronhydrochlorid ist.
